# FASCICULE DE BREVET EUROPEEN

(11) **EP 2 268 336 B1**
(45) Date de publication et mention de la délivrance du brevet: **25.03.2015**
(21) Numéro de dépôt: 09742232.3
(22) Date de dépôt: 02.04.2009
(51) Int. Cl.: A61M 1/10

(54) **NOUVEAU DISPOSITIF MEDICAL PULSATILE**
NEUARTIGES PULSIERENDES MEDIZINISCHES GERÄT
NOVEL MEDICAL PULSATING DEVICE

(30) Priorité: 02.04.2008 FR 0801816
(43) Date de publication de la demande: 05.01.2011
(73) Titulaire: Nour, Sayed, 92370 Chaville (FR); Chastanier, Pierre, 75008 Paris (FR)
(72) Inventeur: Nour, Sayed, 92370 Chaville (FR)
(74) Mandataire: Thomas, Nadine
(86) Numéro de dépôt international: PCT/FR2009/050564
(87) Numéro de publication internationale: WO 2009/136034

(56) Documents cités:
- WO-A-00/35358
- WO-A-2008/000110
- US-A- 4 362 150
- US-A- 4 902 273
- US-A- 5 090 957
- US-A- 5 697 906
- US-B1- 6 733 459

## Description

La présente invention a pour objet un nouveau dispositif médical pulsatile. Conceptuellement, le système cardio-vasculaire est un circuit hydraulique, fermé, sous pression, tapissé intérieurement par des cellules endothéliales. Le fonctionnement de ces cellules endothéliales est régulé par la pulsation cardiaque qui entraîne des variations de pression dans les vaisseaux et donc un cisaillement de ces cellules, ce qui les stimule. Ces forces tangentielles du stress de cisaillement (shear stress) sont indispensables au maintien de la fonction endothéliale comprenant le tonus vasculaire par la synthèse d'oxyde nitrique (NOS), la coagulation du sang, la réponse inflammatoire, l'immunité, l'athérosclérose, l'angiogénèse et l'apoptose. La fonction endothéliale est très importante puisqu'elle contrôle l'embryogenèse, la morphogénèse, l'organogénèse ainsi que le maintien d'un organisme sain.

Toute intervention sur ce circuit, telle que, par exemple, une pathologie ou une opération chirurgicale, entraîne un dysfonctionnement endothélial avec des conséquences pouvant être dramatiques. Dans le domaine de l'assistance circulatoire, de nombreux dispositifs pulsatiles sont actuellement utilisés. Parmi ces dispositifs pulsatiles, le plus utilisé en raison de sa facilité d'utilisation et de son caractère peu onéreux est le ballon de contre-pulsion intra-aortique (IABP : Intra Aortic Balloon Pump). Dans la demande de brevet US 2001/0031907 est divulgué un dispositif pulsatile d'assistance à la circulation sanguine comprenant :
- un cathéter, destiné à être inséré dans un vaisseau sanguin, ayant un diamètre et présentant une tige à son extrémité d'insertion et,
- un élément gonflable logé autour d'une partie du cathéter, augmentant, lorsqu'il est gonflé, la pression diastolique dans les artères coronaires, ledit élément gonflable étant destiné à être relié à un moyen de gonflage, placé du côté de l'autre extrémité dudit cathéter et permettant le gonflage/dégonflage dudit élément gonflable, de manière pulsée.

On connaît le brevet américain US 4 362 150 qui divulgue un cathéter intra-aortique muni à son extrémité distale d'un élément gonflable enroulable sur un axe pour sa pénétration dans l'aorte. L'élément gonflable est fixé à l'une de ses extrémités sur la paroi extérieure distale du cathéter, et à l'autre extrémité sur un embout d'une tige interne flexible elle-même fixée et prolongeant l'extrémité distale du cathéter. Ce cathéter représente l'état de la technique le plus proche.

Le brevet US 6 733 459 concerne un cathéter intra aortique muni d'un ballon gonflable à son extrémité distale ; ce cathéter comprend en outre un système de mesure de la pression sanguine à l'extrémité distale du cathéter.

Le document WO 2008/000110 montre une tubulure pulsatile qui n'est nullement destinée à être insérée dans un vaisseau sanguin ; cette tubulure est intercalée entre une machine extérieure au corps humain et le corps du patient ; elle permet de véhiculer du sang et ne peut donc correspondre au cathéter au sens de l'invention.
Le document WO 2000/35358 divulgue un cathéter comportant un ballon à une de ses extrémités.

Le brevet US 5 090 957 montre un cathéter intra aortique muni à son extrémité distale d'un moyen pour recourber son extrémité munie d'un ballon.

Le brevet US 5 697 906 divulgue un cathéter intra aortique dont le tube interne présente selon sa longueur plusieurs flexibilités différentes.
Le brevet US 4 902 273 enseigne un dispositif d'assistance cardiaque.

Actuellement, lorsque l'on souhaite insérer un cathéter à élément gonflable ou ballon, du type précité, dans un vaisseau sanguin d'un patient, on utilise au préalable un introducteur, constitué d'un tube de guidage et d'une valve hémostatique anti-reflux. L'insertion du tube de guidage crée une ouverture d'un diamètre suffisant pour introduire, par la suite, le cathéter à ballon à l'intérieur dudit tube de guidage tandis que la valve, placée au point d'insertion, permet de réduire et d'arrêter l'écoulement sanguin au travers de l'introducteur.

L'inconvénient présenté par les cathéters à ballon de l'art antérieur est que si l'on souhaite insérer ledit cathéter à ballon non pas au travers d'un introducteur mais directement dans le vaisseau sanguin, comme c'est le cas, par exemple, lorsque l'on souhaite laisser un cathéter à ballon en place pendant un certain temps ou pour des cathéters de faible diamètre (c'est-à-dire de l'ordre de quelques millimètres ou moins), les risques d'écoulement sanguin ne sont pas négligeables. En effet, lors de l'insertion directe du cathéter à ballon, le diamètre du point d'insertion, qui est au départ égal au diamètre du cathéter, se voit agrandi au passage de la partie du cathéter entourée du ballon. Par la suite, lorsque le cathéter est inséré plus en profondeur de sorte à positionner le ballon au niveau de la région à traiter, le diamètre du cathéter se retrouve inférieur au diamètre du point d'insertion, d'où des risques d'écoulement sanguin.

L'invention se propose donc de résoudre ce problème grâce à un dispositif médical pulsatile comprenant :
- un cathéter, destiné à être inséré dans un vaisseau sanguin, ayant un diamètre et présentant une tige à son extrémité d'insertion, dite partie A, et,
- un élément gonflable logé autour d'une partie du cathéter, dite partie B, ledit élément gonflable étant destiné à être relié à un moyen de gonflage placé du côté de l'autre extrémité dudit cathéter, dite partie C, et permettant le gonflage/dégonflage dudit élément gonflable, de manière pulsée
caractérisé en ce que le diamètre de la partie B du cathéter est inférieur aux diamètres des parties A et C du cathéter et en ce que les diamètres des parties A et C dudit cathéter sont sensiblement égaux.

Cette diminution du diamètre de la partie B du cathéter permet que le diamètre de l'ensemble partie B du cathéter plus élément gonflable soit diminué par rapport à celui des cathéters de l'art antérieur. Ainsi, lors de l'insertion du cathéter dans le vaisseau sanguin d'un patient, il n'y aura pas trop d'élargissement dudit point d'insertion dû à un diamètre nettement supérieur d'une partie du cathéter.

Dans un mode de réalisation particulier de l'invention, le diamètre de la partie B du cathéter plus l'élément gonflable à l'état dégonflé est égal ou inférieur aux diamètres des parties A et C du cathéter. Dans ce mode de réalisation, l'insertion du cathéter n'entraîne aucun élargissement du point d'insertion, ce qui est encore plus avantageux.

Dans une forme d'exécution particulière, l'élément gonflable est un ballon.

De préférence, l'élément gonflable est formé d'un matériau radio-opaque, biocompatible. Ainsi, l'insertion de cet élément gonflable dans un vaisseau sanguin n'entraîne aucune infection ou autre dommage et peut facilement être visualisée par radiographie.

Dans une forme d'exécution particulière, ledit matériau est du polyuréthane, ce matériau est un exemple, parmi d'autres, de matériau organique avantageux.

Dans un mode de réalisation particulier de l'invention, le diamètre du cathéter est de quelques millimètres ou moins. Ces dimensions correspondent à de petits cathéters utilisés en pédiatrie mais également chez l'adulte pour atteindre certains petits vaisseaux sanguins. Or, le problème soulevé par l'invention, à savoir la variation de diamètre d'un cathéter à ballon lorsque l'on souhaite l'utiliser sans tube de guidage, est fortement accentué dans le cas de cathéter de faible diamètre. En effet, l'épaisseur du ballon représente pour ces petits cathéters une augmentation de leur diamètre nettement supérieure, en proportion, par rapport aux cathéters de diamètre plus important.

Dans un mode de réalisation particulier de l'invention, les dimensions de l'élément gonflable sont une longueur comprise entre 0,1 et 2 cm et un volume compris entre 0,1 et 2 cm³.

L'invention concerne également un ensemble médical pulsatile comprenant un dispositif pulsatile, tel que décrit ci-dessus, et un moyen de gonflage comprenant :
- une poche, adaptée à être remplie de fluide,
- des moyens de compression de poche, adaptés à comprimer ladite poche de manière pulsée ; et
- un moyen de connexion reliant ladite poche audit élément gonflable du cathéter et permettant la circulation du fluide entre ledit élément gonflable et ladite poche.

Cet ensemble médical pulsatile est simple d'utilisation et peu onéreux. De plus, de par sa taille réduite, il est portable. Les moyens de compression de poche peuvent être manuels ou bien prendre la forme de plaques comprimant la poche par translation ou par rotation des deux plaques, ou par translation de l'une des plaques vers l'autre qui reste fixe, ou encore la forme d'un compartiment dans lequel vient se loger la poche.

Dans une forme d'exécution particulière de l'ensemble médical pulsatile, les moyens de compression de poche sont commandés électromécaniquement.

Dans une forme d'exécution particulière de l'ensemble médical pulsatile, le dispositif pulsatile et le moyen de gonflage sont d'un seul tenant.

Un mode de réalisation de l'invention va maintenant être décrit par référence aux dessins annexés, dans lesquels :
- la figure 1 représente une coupe longitudinale du dispositif pulsatile de l'invention lorsque le ballon est à l'état dégonflé ;
- la figure 2 représente une coupe longitudinale du dispositif pulsatile de l'invention lorsque le ballon est à l'état gonflé ; et
- la figure 3 représente une coupe longitudinale du dispositif pulsatile de l'invention relié à une forme d'exécution particulière de moyen de gonflage.

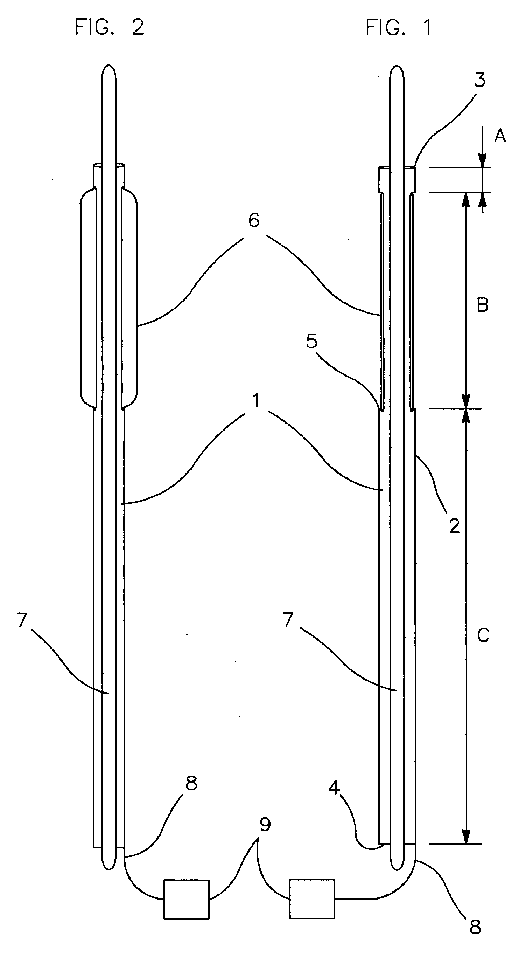

Le dispositif pulsatile de l'invention, représenté aux figures 1, 2 et 3, est constitué d'un cathéter 1, qui est un tube creux, présentant trois parties successives A, B et C. La partie A, également appelée tige, est la première à être insérée dans le vaisseau sanguin du patient. Ledit cathéter 1 présente une paroi externe 2 ainsi que deux extrémités 3 (côté partie A) et 4 (côté partie C). La partie B du cathéter 1 présente un renfoncement 5 dans la paroi externe 2. Ce renfoncement 5 s'étend sur la longueur B et est présent sur l'entière circonférence du cathéter 1. Du fait de la présence de ce renfoncement 5, alors que les parties A et C du cathéter 1 présentent sensiblement le même diamètre, la partie B est, quant à elle, de diamètre inférieur. Un ballon 6, gonflable, est placé de la même manière que dans l'art antérieur, c'est-à-dire, par exemple, par soudage dans le renfoncement 5 du cathéter 1. Le renfoncement 5 est tel que le diamètre de la partie B du cathéter munie du ballon à l'état dégonflé est alors sensiblement égal aux diamètres des parties A et C. A l'intérieur du cathéter 1 est inséré un guide métallique 7. Un port connecteur de fluide 8, intégré dans la paroi du cathéter 1, met en communication le ballon gonflable 6 et un moyen de gonflage 9 représenté schématiquement, sur les figures 1 et 2, du côté de l'extrémité 4, ce moyen de gonflage pouvant être une console.

A la figure 1, le ballon 6 est à l'état dégonflé, de ce fait le diamètre de la partie B du cathéter 1 avec le ballon 6 à l'état dégonflé est sensiblement égal au diamètre des parties A et C du cathéter 1.

A la figure 2 le ballon 6 est à l'état gonflé, le diamètre de la partie B du cathéter 1 avec le ballon 6 à l'état gonflé est alors supérieur au diamètre des parties A et C du cathéter 1.

L'insertion du dispositif pulsatile de l'invention va maintenant être décrite. On pique un vaisseau sanguin du patient avec une aiguille ce qui crée une ouverture, ou point d'insertion. Le guide métallique 7 est alors mis en place. Puis le cathéter 1 est inséré au travers de cette ouverture grâce au guide 7. Les parties A, B et C du cathéter 1 passent successivement au travers de cette ouverture, n'entraînant aucun élargissement de ladite ouverture puisque, comme dit plus haut, les parties A et C et la partie B, munie du ballon 6 à l'état dégonflé, ont sensiblement le même diamètre. Une fois que le cathéter 1 est en place, c'est-à-dire une fois que le ballon 6 occupe la région du vaisseau sanguin devant être traitée, le point d'insertion est alors parfaitement comblé par la partie C du cathéter et l'écoulement sanguin est ainsi réduit voire annulé.

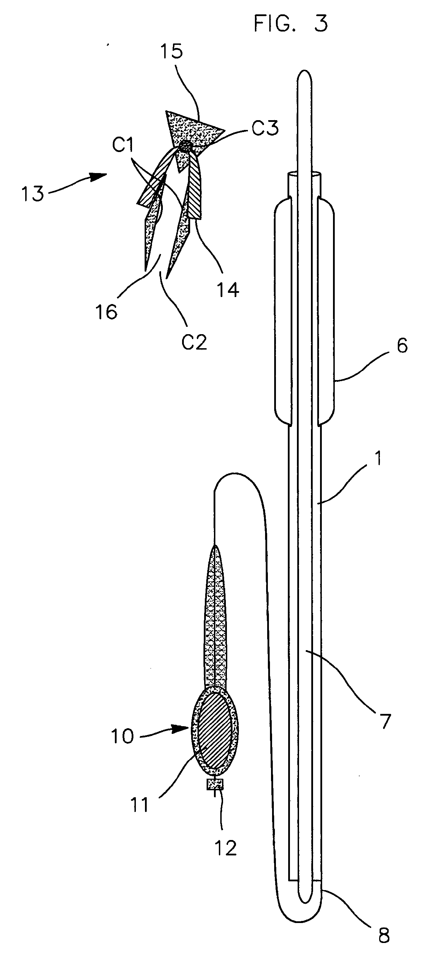

La figure 3 représente le cathéter 1 de la figure 2 relié à une forme d'exécution particulière de moyen de gonflage 9.

Ce moyen de gonflage 9 comprend :
- une première partie incluant une poche 10 remplie de fluide 11, reliée à une extrémité au port connecteur de fluide 8 et à son autre extrémité à une valve 12 anti-reflux, et
- une deuxième partie incluant un moyen de compression 13 de ladite poche 10 comprenant un compartiment compresseur de poche 14 et une commande 15, par exemple, électromécanique dudit compartiment compresseur 14. Le compartiment compresseur de poche est schématisé sous une forme grossièrement rectangulaire avec deux grands côtés C1 et l'un des petits côtés C2 ouvert tandis que le second C3 est relié à la commande électromécanique 15 représentée schématiquement par un triangle. Le compartiment compresseur 14 présente donc un évidemment 16.

On place le cathéter 1 de l'invention dans un vaisseau sanguin d'un patient, comme décrit ci-dessus. L'extrémité du port connecteur de fluide 8 faisant saillie hors du corps du patient est connectée à la poche 10. La poche 10 est alors remplie de fluide 11 (qui peut être de l'hélium, du dioxyde de carbone, du sérum physiologique) par ouverture de la valve 12 (cette opération pouvant avoir été effectuée avant la connexion du port connecteur de fluide 8 à la poche 10). La poche 10 est ensuite placée dans l'évidemment 16 du compartiment compresseur 14 commandé par la commande électromécanique 15. Selon les instructions reçues par ladite commande électromécanique 15, un rythme précis de pression/décompression de la poche 10 se met en place, ce rythme pouvant être, par exemple, de 10 à 300 pressions par minute. La compression de la poche 10 entraîne un flux de fluide 11 en direction du ballon 6 qui se gonfle et la décompression de la poche 10 entraîne un appel de fluide 11 du ballon 6 vers la poche 10, ce qui dégonfle le ballon. Un mouvement de pulsation du fluide est ainsi transmis de la poche 10 jusqu'au ballon 6. Cet ensemble cathéter-ballon-moyen de gonflage (tel que décrit à la figure 3) forme donc un ensemble médical pulsatile portable. En effet, le moyen de gonflage comprenant la poche 10, le compartiment compresseur de poche 14 et la commande électromécanique 15, est facilement transportable par le patient lors de ses déplacements, ce qui lui permet de garder une certaine mobilité.

Ce moyen de gonflage est de faible coût et d'utilisation simple. En effet, la compression/décompression ne nécessite pas de source de pression coûteuse au contraire des consoles des ballons intra-aortiques de l'art antérieur.

Les dispositifs pulsatiles de l'invention pouvant s'appliquer aux cathéters de petit diamètre, on disposera de petits cathéters pulsatiles, ce qui n'est actuellement pas le cas. Ces petits cathéters pulsatiles pourront être utilisés dans de nombreuses applications.

En effet, l'inventeur a découvert, de façon tout à fait inattendue, que le gonflement du ballon, placé dans un vaisseau sanguin, augmente la force de cisaillement sur la paroi du vaisseau sanguin.

Ces petits cathéters pulsatiles pourront donc être utilisés non seulement pour traiter des artères coronaires bouchées (le petit diamètre du cathéter permettant d'atteindre la région bouchée de l'artère et le mouvement pulsatile du ballon permettant de traiter de manière douce ladite région), mais également pour traiter de possibles malformations du foetus en passant par les vaisseaux ombilicaux. De nombreuses autres applications sont envisageables comme dans l'interdépendance angiogénèse-apoptose (par exemple, augmenter l'angiogénèse en cas de fracture chez une personne agée afin d'accélérer la cicatrisation), l'athérosclérose (coronaire, cérébrale, rénale), le système immun, la cardiogénèse, la sécrétion de monoxyde d'azote (pour traiter, par exemple, l'hypertension artérielle systémique ou pulmonaire aiguë et surtout chronique).

## Revendications

1. Dispositif médical pulsatile comprenant :
- un cathéter (1), destiné à être inséré dans un vaisseau sanguin, ayant un diamètre et présentant :
- une extrémité d'insertion (3), dite partie A, comportant une tige ;
- une extrémité (4), dite partie C, opposée ladite partie A, de diamètre sensiblement égal au diamètre de la partie A ;
- une partie B située entre ladite partie A et ladite partie C, présentant un renfoncement (5) dans sa paroi externe (2), ce renfoncement étant présent sur l'entière circonférence du cathéter et tel que le diamètre de la partie B dudit cathéter (1) est inférieur aux diamètres des parties A et C dudit cathéter (1) ;
- un élément gonflable (6), logé autour d'une partie du cathéter (1) et placé dans ledit renfoncement (5) du cathéter (1), ledit élément gonflable (6) étant destiné à être relié à un moyen de gonflage (9), placé du côté de ladite partie C, et permettant le gonflage/dégonflage dudit élément gonflable, de manière pulsée ;
**caractérisé en ce que** lesdites parties A, B, C sont constituées par un tube creux.

2. Dispositif médical pulsatile selon la revendication 1 **caractérisé en ce que** le diamètre de la partie B dudit cathéter (1) plus l'élément gonflable (6) à l'état dégonflé est égal ou inférieur aux diamètres des parties A et C dudit cathéter (1).

3. Dispositif médical pulsatile selon la revendication 1 ou 2, **caractérisé en ce que** ledit élément gonflable (6) est un ballon.

4. Dispositif médical pulsatile selon la revendication 1, 2 ou 3, **caractérisé en ce que** ledit élément gonflable (6) est formé d'un matériau radio- opaque, biocompatible.

5. Dispositif médical pulsatile selon la revendication 4, **caractérisé en ce que** ledit matériau est du polyuréthane.

6. Dispositif médical pulsatile selon l'une quelconque des revendications précédentes, **caractérisé en ce que** les dimensions de l'élément gonflable (6) sont une longueur comprise entre 0,1 et 2 cm et un volume compris entre 0,1 et 2 cm3.

7. Ensemble médical pulsatile comprenant un dispositif pulsatile selon l'une quelconque des revendications précédentes et un moyen de gonflage (9) comprenant :
- une poche (10), adaptée à être remplie de fluide (11),
- des moyens de compression de poche (14) adaptés à comprimer ladite poche (10) de manière puisée ; et
- un moyen de connexion (8) reliant ladite poche (10) audit élément gonflable (6) du cathéter et permettant la circulation du fluide (11) entre ledit élément gonflable (6) et ladite poche (10).

8. Ensemble médical pulsatile selon la revendication 7, **caractérisé en ce que** lesdits moyens de compression de poche (14) sont commandés (15) électromécaniquement.

9. Ensemble médical pulsatile selon la revendication 7 ou 8, **caractérisé en ce que** le dispositif pulsatile et le moyen de gonflage sont d'un seul tenant.

## Patentansprüche

1. Pulsierende medizinische Vorrichtung, die Folgendes umfasst:
- einen Katheter (1), der dazu vorgesehen ist, in ein Blutgefäß eingeführt zu werden, einen Durchmesser hat und Folgendes aufweist:
- ein Einführende (3), der so genannte Teil A, das eine Stange umfasst;
- ein Ende (4), der so genannte Teil C, das dem besagten Teil A gegenüberliegt, mit einem Durchmesser, der im Wesentlichen gleich dem Durchmesser des Teils A ist;
- einen Teil B, der sich zwischen dem besagten Teil A und dem besagten Teil C befindet und eine Verstärkung (5) in seiner Außenwand (2) aufweist, wobei diese Verstärkung auf dem gesamten Umfang des Katheters vorliegt und der Durchmesser des Teils B des besagten Katheters (1) kleiner als die Durchmesser der Teile A und C des besagten Katheters (1) ist;
- ein aufblasbares Element (6), das um einen Teil des Katheters (1) herum untergebracht ist und in der besagten Verstärkung (5) des Katheters (1) angeordnet ist, wobei das besagte aufblasbare Element (6) dazu vorgesehen ist, mit einem Aufblasmittel (9) verbunden zu werden, das an der Seite des besagten Teils C angeordnet ist und das Aufblasen/Entleeren des besagten aufblasbaren Elements auf pulsierte Weise ermöglicht;
**dadurch gekennzeichnet, dass** die besagten Teile A, B, C von einem hohlen Rohr gebildet werden.

2. Pulsierende medizinische Vorrichtung nach Anspruch 1, **dadurch gekennzeichnet, dass** der Durchmesser des Teils B des besagten Katheters (1) zuzüglich des aufblasbaren Elements (6) im entleerten Zustand kleiner gleich den Durchmessern der Teile A und C des besagten Katheters (1) ist.

3. Pulsierende medizinische Vorrichtung nach Anspruch 1 oder 2, **dadurch gekennzeichnet, dass** das besagte aufblasbare Element (6) ein Ballon ist.

4. Pulsierende medizinische Vorrichtung nach Anspruch 1, 2 oder 3, **dadurch gekennzeichnet, dass** das besagte aufblasbare Element (6) aus einem röntgendichten, biokompatiblen Material hergestellt ist.

5. Pulsierende medizinische Vorrichtung nach Anspruch 4, **dadurch gekennzeichnet, dass** das besagte Material Polyurethan ist.

6. Pulsierende medizinische Vorrichtung nach einem der vorhergehenden Ansprüche, **dadurch gekennzeichnet, dass** die Dimensionen des aufblasbaren Elements (6) eine Länge zwischen 0,1 und 2 cm und ein Volumen zwischen 0,1 und 2 cm³ sind.

7. Pulsierende medizinische Einheit, die eine pulsierende Vorrichtung nach einem der vorhergehenden Ansprüche und ein Aufblasmittel (9) umfasst, das Folgendes umfasst:
- einen Beutel (10), der dazu geeignet ist, mit Fluid (11) gefüllt zu werden,
- Beutelzusammendrückmittel (14), die dazu geeignet sind, den besagten Beutel (10) auf pulsierte Weise zusammenzudrücken; und
- ein Verbindungsmittel (8), das den besagten Beutel (10) mit dem aufblasbaren Element (6) des Katheters verbindet und die Zirkulation des Fluids (11) zwischen dem besagten aufblasbaren Element (6) und dem besagten Beutel (10) ermöglicht.

8. Pulsierende medizinische Einheit nach Anspruch 7, **dadurch gekennzeichnet, dass** die besagten Beutelzusammendrückmittel (14) elektromechanisch betätigt (15) werden.

9. Pulsierende medizinische Einheit nach Anspruch 7 oder 8, **dadurch gekennzeichnet, dass** die pulsierende Vorrichtung und das Aufblasmittel einstückig sind.

## Claims

1. Pulsating medical device comprising:
- a catheter (1), to be inserted into a blood vessel, having a diameter and having:
- one insertion end (3), referred to as part A, comprising a rod and;
- one end (4), referred to as part C, opposite said part A, of a diameter substantially equal to the diameter of the part A;
- one part B situated between the part A and said part C, having a recess (5) in its external face (2), this recess being present on the entire circumference of the catheter and such that the diameter of the portion B of said catheter (1) is less than the diameter of parts A and C of said catheter (1);
- an inflatable component (6), housed around a part of the catheter (1), and positioned in the said recess (5) of catheter (1), the said inflatable component (6) being designed to be connected to an inflation means (9) positioned on the side of the said part C, and allowing the inflation/deflation of said inflatable component, in a pulsed manner;
**characterised in that** the said parts A, B C are constituted by an hollow pipe.

2. Pulsating medical device according to claim 1, **characterised in that** the diameter of part B of said catheter (1) plus the deflated inflatable component (6) at the deflation state is equal or less than the diameters of parts A and C of said catheter (1).

3. Pulsating medical device according to claim 1 or 2, **characterised in that** said inflatable component (6) is a balloon.

4. Pulsating medical device according to claim 1, 2 or 3, **characterised in that** said inflatable component (6) is formed from a biocompatible radio-opaque material.

5. Pulsating medical device according to claim 4, **characterised in that** said material is polyurethane.

6. Pulsating medical device according to any of the above claims, **characterised in that** the dimensions of the inflatable component (6) are a length between 0.1 and 2 cm and a volume between 0.1 and 2 cm³.

7. Pulsating medical assembly comprising a pulsating device according to any of the above claims and inflation means (9) comprising:
- a bag (10), suitable for being filled with fluid (11),
- bag compression means (14), suitable for compressing said bag (10) in a pulsed manner; and
- connection means (8) connecting said bag(10) to said inflatable component (6) of the catheter, enabling the circulation of the fluid (11) between said inflatable component(6) and said bag (10).

8. Pulsating medical assembly according to claim 7, **characterised in that** said bag compression means (14) are controlled (15) electromechanically.

9. Pulsating medical assembly according to claim 7 or 8, **characterised in that** the pulsating device and the inflations means are integral.
